(19) Europäisches Patentamt / European Patent Office / Office européen des brevets

(11) EP 3 442 700 B1

(12) # EUROPÄISCHE PATENTSCHRIFT

(45) Veröffentlichungstag und Bekanntmachung des Hinweises auf die Patenterteilung:
**20.05.2020 Patentblatt 2020/21**

(21) Anmeldenummer: **17719803.3**

(22) Anmeldetag: **05.04.2017**

(51) Int Cl.:
***B01J 20/24*** *(2006.01)* ***B01J 20/26*** *(2006.01)*
***B01J 20/288*** *(2006.01)* ***B01J 20/289*** *(2006.01)*
***B01J 20/32*** *(2006.01)* ***B01D 15/38*** *(2006.01)*
***C07K 1/16*** *(2006.01)*

(86) Internationale Anmeldenummer:
**PCT/EP2017/000430**

(87) Internationale Veröffentlichungsnummer:
**WO 2017/178100 (19.10.2017 Gazette 2017/42)**

(54) **MULTIMODALES ADSORPTIONSMEDIUM MIT MULTIMODALEN LIGANDEN, VERFAHREN ZU DESSEN HERSTELLUNG UND DESSEN VERWENDUNG**

MULTIMODAL ADSORPTION MEDIUM WITH MULTIMODAL LIGANDS, METHOD FOR THE PREPARATION AND USE THEREOF

ADSORBANT MULTIMODAL COMPORTANT DES LIGANDS MULTIMODAUX, SON PROCÉDÉ DE PRÉPARATION ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.04.2016 DE 102016004432**

(43) Veröffentlichungstag der Anmeldung:
**20.02.2019 Patentblatt 2019/08**

(73) Patentinhaber: **Sartorius Stedim Biotech GmbH**
**37079 Göttingen (DE)**

(72) Erfinder:
- **KUPRACZ, Lukas**
  **30173 Hannover (DE)**
- **TAFT, Florian**
  **37085 Göttingen (DE)**
- **VILLAIN, Louis**
  **30451 Hannover (DE)**
- **KUPER, Kornelia**
  **37085 Göttingen (DE)**

(74) Vertreter: **Müller-Boré & Partner**
**Patentanwälte PartG mbB**
**Friedenheimer Brücke 21**
**80639 München (DE)**

(56) Entgegenhaltungen:
**EP-A1- 0 316 492**
**WO-A2-2006/081002**
**WO-A2-2012/151352**
**GB-A- 2 232 984**
**US-A- 3 951 815**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 442 700 B1

Printed by Jouve, 75001 PARIS (FR)

## Beschreibung

Die vorliegende Erfindung betrifft ein multimodales Adsorptionsmedium, insbesondere ein multimodales Chromatographiemedium, ein Verfahren zu dessen Herstellung, sowie die Verwendung des erfindungsgemäßen Adsorptionsmediums bzw. eines erfindungsgemäß hergestellten Adsorptionsmediums zur Aufreinigung von Biomolekülen.

Als "Adsorptionsmedium" werden Adsorbentien bezeichnet, die über funktionelle Oberflächengruppen, nachfolgend auch als "Liganden" und/oder "chromatographisch aktive Zentren" bezeichnet, verfügen, die mit bestimmten Komponenten von Fluiden selektiv Bindungen eingehen können. Zielsubstanz(en) und/oder Kontaminant(en) werden erfindungsgemäß als "Adsorbenden" bezeichnet, wobei es sich auch um mehrere unterschiedliche Substanzen handeln kann. Adsorbenden können Einzelmoleküle, Assoziate oder Partikel sein, bei denen es sich vorzugsweise um Proteine oder andere Substanzen biologischen Ursprungs handelt.

Die Bindung der Adsorbenden an das Adsorbens kann reversibel oder irreversibel sein, in jedem Fall ermöglicht sie ihre Abtrennung von den Fluiden, bei denen es sich beispielsweise um wässrige Flüssigkeiten handelt und die im Folgenden "Medien" genannt werden. Unter dem Begriff "Elution" werden die Desorption und die damit einhergehenden Spülschritte etc. zusammengefasst, und das zur Elution verwendete Medium ist das "Eluens". Die Komponenten können eine oder mehrere Zielsubstanzen und/oder eine oder mehrere Kontaminanten darstellen. "Zielsubstanzen" sind Wertstoffe, die aus dem Medium in angereicherter oder reiner Form gewonnen werden sollen. Zielsubstanzen können beispielsweise rekombinante Proteine, wie z.B. monoklonale Antikörper sein. "Kontaminanten" sind Stoffe, deren Abwesenheit oder Entfernung aus dem Fluid aus technischen, regulatorischen oder sonstigen Gründen erforderlich oder wünschenswert ist. Kontaminanten können beispielsweise Viren, Proteine, Aminosäuren, Nukleinsäuren, Endotoxine, Proteinaggregate, Liganden oder deren Teile sein. Für die Entfernung von Kontaminanten, die als "negative Adsorption" bezeichnet wird, kann (darf) die Adsorption irreversibel verlaufen, wenn das Adsorbens nur einmal verwendet werden soll. Bei der Adsorption der Zielsubstanz(en) muss der Vorgang reversibel verlaufen. Es kann entweder eine bloße Anreicherung oder eine Auftrennung in mehrere Zielsubstanzen durchgeführt werden, wobei im letzteren Fall entweder die Adsorption, die Desorption oder beide selektiv erfolgen können.

Den Vorgang bezeichnet man als adsorptive Stofftrennung oder Chromatographie. Herkömmliche Adsorbentien für die Chromatographie sind entweder partikulär geformt und werden in Form von Schüttungen in Säulen betrieben, oder liegen in Form von Adsorptionsmembranen vor, die sich meist in Modulen befinden, deren Bauformen den in der Membranfiltration üblichen entsprechen (z.B. Wickelmodul, Stapelmodul etc.). Allen Adsorbentien ist üblicherweise die Grundanforderung nach möglichst niedriger unspezifischer Adsorption gemeinsam.

Im Stand der Technik ist eine Vielzahl von synthetischen und natürlichen Liganden bekannt. Der Bindung des Liganden an den Träger kann eine "Aktivierung" des Trägers vorausgehen, also die Einführung von reaktiven, funktionellen Gruppen, die zur spontanen Bindung des Liganden befähigt sind. Seltener weist der Ligand selbst eine reaktive Gruppe auf, wie z.B. die als Farbstoffliganden dienenden Reaktivfarbstoffe aus der Textilindustrie. Methoden zur Bindung von funktionellen Gruppen sind dem Fachmann an sich bekannt (z.B. aus Greg T. Hermanson, A. Krishna Mallia, Paul K. Smith, Immobilized Affinity Ligand Techniques, Academic Press, INC, 1992).

Die Filtration, Aufreinigung oder Entfernung von Biomolekülen wie Proteinen, Aminosäuren, Nukleinsäuren, Viren oder Endotoxinen aus flüssigen Medien ist von großem Interesse für die biopharmazeutische Industrie. Die meisten Anwendungen der Kontaminantenentfernung werden zurzeit mit konventionellen Chromatographiegelen oder Chromatographiemembranen betrieben.

Dabei erlangte die Ionenaustauschchromatographie eine wichtige Stellung in der Aufreinigung von Biomolekülen. Kationenaustauscher, in denen Mischmodus- oder multimodale Liganden vorhanden sind, sind bereits seit längerem im Stand der Technik bekannt. Beispielsweise offenbart US 5 431 807 A ein multimodales chromatographisches Trennungsmedium, bei welchem hydrophobe Liganden, wie beispielweise Benzyl-Liganden, in Poren eines ersten Größenbereichs fixiert werden, während ionenaustauschende Liganden in Poren eines zweiten Größenbereichs räumlich separiert von den hydrophoben Liganden fixiert sind. In ähnlicher Weise wird in EP 0 665 867 B1 ein Verfahren zur porengrößenselektiven chemischen Modifizierung poröser Materialien beschrieben, wobei beispielsweise hydrophobe Liganden in Poren eines ersten Größenbereichs fixiert werden, während kationenaustauschende Liganden in Poren eines zweiten Größenbereichs räumlich separiert von den hydrophoben Liganden fixiert werden. US 2012/0202976 A1 offenbart ein chromatographisches Trennungsmedium, bei welchem sowohl eine erste Art hydrophober Liganden als auch eine zweite Art ionenaustauschender Liganden, letztere über sog. Extender, an die Chromatographiematrix gebunden sind. Bei diesen Systemen liegen die unterschiedlichen Funktionalitäten folglich in unterschiedlichen Molekülketten und räumlich separiert vor.

Ein Nachteil bekannter Kationenaustauscher ist, dass sie nur unter verhältnismäßig geringen ionischen Stärken Substanzen binden können, so dass häufig ein Verdünnen des Mediums vor der Adsorption erfolgen muss. Die bekannten Adsorptionsmedien tolerieren deshalb keine hohen Salzkonzentrationen bei der Bindung der Substanzen, wodurch ein zusätzlicher Verdünnungsschritt und das Arbeiten mit großen Fluidvolumina in entsprechend groß ausgelegten Chromatographieanlagen notwendig werden.

In US 8 877 904 B2 werden Chromatographiematrizen offenbart, auf denen multimodale Liganden mit kationenaustauschenden und hydrophoben Funktionalitäten fixiert sind, wobei beispielsweise Liganden ausgehend von Phenylalanin oder 6-Aminohexansäure an die Oberflächen eines Trägermaterials gebunden werden. US 8 017 740 B2 offenbart Chromatographiematrizen auf Basis poröser Formkörper, vorzugsweise abgeleitet von anorganischen Hydroxy- oder Fluorapatiten, auf denen sowohl hydrophobe als auch kationenaustauschende Liganden fixiert sind, wobei beispielsweise Capto™ MMC genannt wird, ein sogenannter "mixed mode" Ligand. Vergleichbare Chromatographiematrizen mit diesem Capto™ MMC Ligand werden auch in US 2013/0109807 A1 offenbart. Der Capto™ MMC Ligand weist einen 2-Benzoylamino-butansäurerest auf, welcher durch Umsetzung von Homocysteinthiolacton mit Benzoylchlorid und anschließende Thiolactonöffnung erhalten wird. Die Anbindung des Capto™ MMC Liganden an die stationäre Phase erfolgt dann über die Thiolgruppe mittels nukleophiler Substitution oder Ringöffnung.

US 6 852 230 B2 offenbart Chromatographiematrizen, die Liganden mit kationenaustauschenden und hydrophoben Gruppen aufweisen, wobei die Matrizen eine hohe Wiedergewinnungsrate von bovinem Serumalbumin unter hohen Salzkonzentrationen ermöglichen. Diese multimodalen Systeme werden genauer in der zugrunde liegenden Publikation B.-L. Johansson et al., Journal of Chromatography A, 1016 (2003), 35-49, beschrieben, wobei Liganden auf aktivierte "Sepharose™ 6 Fast Flow" anhand zweier Varianten immobilisiert werden. Über eine mehrstufige Synthese wird hierbei zunächst Mercaptopropionsäure an den aktivierten Träger gebunden. Anschließend wird die Säurefunktion mit Dicyclohexylcarbodiimid (DCC) und *N*-Hydroxysuccinimid (NHS) aktiviert und der resultierende Ester wird schließlich mit einem Aminosäurederivat umgesetzt, um eine entsprechende kationenaustauschende Funktionalität einzuführen.

WO 2006/081002 A2 offenbart multimodale Adsorptionsmedien für die chromatographische Trennung von Biomolekülen, umfassend ein polymeres Trägermaterial T aus einem Methacrylat-Polymer, an welches multimodale Liganden der Struktur $-G-CO_2H$ (mit G = $C_2H_4$, CH=CH und $CH(SO_3)-CH_2$) über eine Gruppe -X-(C=O) mit X = NH gebunden sind. Die multimodalen Liganden sind mittels einer Amidbindung über Polyethyleniminketten an der Oberfläche des Trägermaterials T gebunden.

Daraus ergibt sich für die im Stand der Technik bekannten Systeme, dass deren Herstellung im Allgemeinen aufwendige Schritte beinhaltet, nämlich:

i) Das Einbringen einer reaktiven Gruppe auf einem chromatographischen Träger und deren optionale Aktivierung.
ii) Das Umsetzen des daraus hervorgehenden modifizierten Trägers mit einer Thiolverbindung, die eine Säurefunktion enthält.
iii) Das Aktivieren der acidischen Funktionen des festen Trägers mit einem geeigneten Reagens (z.B. N-Hydroxysuccinimid (NHS)) bei Vorhandensein von Dicyclohexylcarbodiimid (DCC)) in einem organischen Lösungsmittel.
iv) Das Hinzugeben eines Aminosäurederivats, das einen geeigneten Rest R umfasst, um einen fertiggestellten Liganden zu erzeugen, der an einen Träger gebunden ist.

So umfasst eine aus dem Stand der Technik bekannte Synthese von Capto™ MMC Matrizen in Schritt i) die Umsetzung von beispielsweise Sepharose mit Allylglycidylether und die nachfolgende Aktivierung des resultierenden Produkts mit Brom, wobei nachfolgend in Schritt ii) die Umsetzung mit einem Thiolacton erfolgt.

Bei diesem Verfahren erfolgt die Umsetzung insbesondere des letzten Schrittes nicht vollständig, wodurch immer ein Produkt mit tatsächlich zwei verschiedenen Liganden erhalten wird, welches einen Thioether-Linker, der eine Säurefunktion enthält und sich aus fehlgeschlagener Umsetzung in Schritt (iii) oder (iv) ergibt, und das gewünschte Endprodukt enthält. Ein chromatographischer Träger, der aus einer Mischung zweier unterschiedlicher Liganden besteht, kann mehrere Probleme verursachen, wenn dieser in einem Trennungsverfahren verwendet wird. Da das Verhältnis der unterschiedlichen Liganden von Charge zu Charge schwanken kann, besitzen die so hergestellten Chromatographiemedien Chargen-abhängig unterschiedliche Eigenschaften, so dass einmal entwickelte Trennmethoden bei Wechsel der Charge nicht reproduzierbar sind. Außerdem kann es bei Verwendung unterschiedlicher separater Liganden zu dem Nachteil einer inhomogenen Verteilung der verschiedenen Liganden auf dem Chromatographiemedium kommen.

Ein weiteres Problem, das mit dem herkömmlichen, oben beschriebenen Verfahren einhergeht, besteht darin, dass häufig keine ausreichend hohen Ligandendichten erhalten werden können, was insbesondere für die Bindungskapazität von kleinen Proteinen nachteilig ist. Dieses Problem kann zumindest teilweise durch polymere Abstandshalter gelöst werden, die auf der Oberfläche der Chromatographiematrix fixiert werden und über welche zusätzliche Liganden an die Matrix gebunden werden können.

Der Erfindung liegt daher die Aufgabe zugrunde, ein Adsorptionsmedium bereitzustellen, welches auf einfache und reproduzierbare Weise hergestellt werden kann, und welches eine hohe Bindungskapazität aufweisen soll, die über einen weiten Salzkonzentrationsbereich gezielt eingestellt werden kann.

Diese Aufgabe wird durch die in den Ansprüchen gekennzeichneten Ausführungsformen der vorliegenden Erfindung gelöst.

Erfindungsgemäß wird ein multimodales Adsorptionsmedium, insbesondere ein multimodales Chromatographiemedium, bereitgestellt, umfassend ein polymeres Trägermaterial T, an welches multimodale Liganden der folgenden

Struktur -G-$(CO_2H)_n$ über eine Gruppe -X-(C=O) kovalent gebunden sind

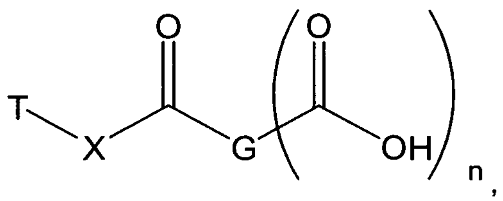

wobei X -NR-, -O- oder -S- und R Alkyl, Alkenyl, Aryl, Heteroaryl oder Wasserstoff darstellen, wobei G eine verzweigte oder unverzweigte $C_{3-10}$ Alkenylgruppe darstellt, die ein oder mehrere Heteroatome, ausgewählt aus O, S, N und Halogenen, enthalten kann und gegebenenfalls mindestens einen Hydroxyl-, Carbonyl-, Carboxyl-, Carbonsäureanhydrid- oder aromatischen Substituenten enthalten kann, und wobei n 1 ist.

Unter dem Begriff "multimodal" wird im Sinne der vorliegenden Erfindung verstanden, dass die Liganden zwei oder mehrere verschiedene Funktionalitäten aufweisen, wodurch diese aufgrund unterschiedlicher chemischer Mechanismen Wechselwirkungen zu den Zielmolekülen eingehen, so dass letztere an das Adsorptionsmedium gebunden werden. Erfindungsgemäß weist das multimodale Adsorptionsmedium bzw. die multimodalen Liganden zumindest kationenaustauschende Säuregruppen und gleichzeitig hydrophobe Gruppen auf. Durch Wahl der hydrophoben Gruppe G können zusätzlich weitere Funktionalitäten integriert werden, die weitere Wechselwirkungen, wie beispielsweise thiophile Wechselwirkungen, $\pi$-$\pi$-Wechselwirkungen, Ionenaustausch-Wechselwirkungen oder Wasserstoffbrückenbindungen, mit Zielsubstanzen eingehen können.

Vorzugsweise weist das erfindungsgemäße Adsorptionsmedium nur eine Art des multimodalen Liganden auf, d.h. dass ausschließlich identische Liganden der vorstehenden Struktur an das polymere Trägermaterial gebunden sind. Indem die multimodale Ligandenstruktur mit Hilfe von nur einem Reagenz erzeugt wird, wie nachfolgend genauer beschrieben wird, ist auf dem erfindungsgemäßen Adsorptionsmedium das Verhältnis von Carbonsäuregruppen zu den Gruppen G, die weitere Wechselwirkungen ermöglichen, stets konstant. Dadurch ist das Ergebnis des Herstellungsprozesses im Vergleich zu einem Modifizierungsverfahren mit zwei unterschiedlichen Liganden oder unvollständigen Umsetzungen stets reproduzierbar.

Bei einer besonders bevorzugten Ausführungsform des erfindungsgemäßen Adsorptionsmediums ist die Gruppe G eine hydrophobe Gruppe, welche über Vander-Waals- oder $\pi$-$\pi$-Wechselwirkungen Bindungen mit den Zielsubstanzen eingehen kann.

Die Herstellung des erfindungsgemäßen Adsorptionsmediums beruht auf einem neuartigen Modifizierungsprotokoll, bei welchem als Ausgangsmaterial ein polymeres Trägermaterial mit mindestens einer Gruppe -XH mit X = -NR-, -O- oder -S- und R = Alkyl, Alkenyl, Aryl, Heteroaryl oder H verwendet wird, welche mit Carbonsäurederivaten unter Ausbildung einer kovalenten Bindung -X-(C=O) reaktionsfähig ist. Die Gruppen -XH werden mit einer Ligandenvorstufe umgesetzt, so dass die resultierenden Liganden über eine kovalente Bindung -X-(C=O) an das Trägermaterial gebunden sind und jeweils über mindestens eine freie Carbonsäuregruppe verfügen. Vorzugsweise werden die Gruppen -XH mit Carbonsäureanhydriden funktionalisiert, wie dies anhand des nachfolgenden Schemas illustriert werden kann:

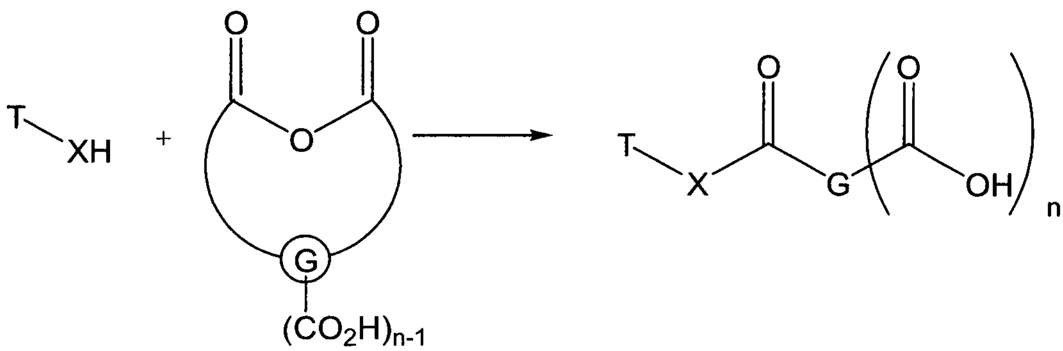

,

wobei X, G und n wie vorstehend definiert sind. Bei den Gruppen G, die selber keine Carbonsäuregruppen -COOH als Substituenten aufweisen, resultiert die einzige im Liganden G-$(CO_2H)_n$ vorhandene Carbonsäuregruppe aus der Ringöffnungsreaktion des Carbonsäureanhydrids mit der nucleophilen Gruppe -XH und n ist gleich 1.

Bei den Gruppen G, die selber mindestens eine Carbonsäuregruppe -COOH als Substituenten aufweisen, ist n nicht erfindungsgemäß größer oder gleich 2, wobei eine der Carbonsäuregruppen aus der Ringöffnungsreaktion des

Carbonsäureanhydrids mit der nucleophilen Gruppe -XH resultiert.

Besonders bevorzugt handelt es sich bei den Gruppen G um hydrophobe Gruppen.

Gemäß einer Ausführungsform der vorliegenden Erfindung ist die Gruppe -X-(C=O) gleich -NH-(C=O). D.h. es handelt sich bei der Gruppe -XH um eine primäre Aminogruppe, so dass die resultierenden Liganden -G-$(CO_2H)_n$ über eine sekundäre Amidbindung -NH-(C=O) an das Trägermaterial gebunden sind.

Als polymeres Trägermaterial eignet sich erfindungsgemäß jegliches Material, das für chromatographische Verfahren als Trägermaterial der stationären Phase verwendet werden kann. Das polymere Trägermaterial, das auch als Chromatographiematrix bezeichnet werden kann, unterliegt keiner besonderen Einschränkung, solange es an seiner Oberfläche Gruppen -XH mit X = -NR-, -O- oder -S- und R = Alkyl, Alkenyl, Aryl, Heteroaryl oder H aufweist, an welche die multimodalen Liganden gebunden werden können bzw. gebunden sein können. Diese Gruppen können auf der Oberfläche des Trägermaterials bereits vorhanden sein, oder auf geeignete Weise eingeführt werden. Demzufolge kann erfindungsgemäß entweder ein polymeres Trägermaterial eingesetzt werden, das originär funktionelle Gruppen aufweist (beispielsweise Polyesterfasern), oder ein polymeres Trägermaterial, bei dem durch eine dem Fachmann bekannte Oberflächenmodifikation geeignete funktionelle Gruppen eingeführt wurden. Bekannte Oberflächenmodifikationen sind in diesem Zusammenhang beispielsweise Substitutions- und Additionsreaktionen, eine Umsetzung mit funktionellen Epoxiden, aktivierten Säuren oder Aktivestern, eine Aktivierung mittels Plasmabehandlung, e-Beam (Elektronenstrahlbehandlung), Gamma-Bestrahlung, Beschichtung, Hydrolyse, Aminolyse, Oxidation, Reduktion, Umsetzung mit funktionellen Carbenen und/oder Nitrenen usw.

Gemäß einer Ausführungsform der vorliegenden Erfindung umfasst das polymere Trägermaterial mindestens ein Material, ausgewählt aus der Gruppe, bestehend aus natürlichen oder synthetischen Fasern, (Polymer)-Membranen, porösen, polymeren monolithischen Formkörpern, Polymergelen, Folien, Vliesen und Geweben.

Beispielhaft für natürliche oder synthetische Fasern, die als Material für das polymere Trägermaterial des erfindungsgemäßen Adsorptionsmediums verwendet werden können, können Polyesterfasern (beispielsweise "Winged Fibers" der Firma Allasso Industries aus Polyethylenterephthalat (PET) oder "4DG™ Fasern" der Firma Fiber Innovation Technology aus Polyethylenterephthalat) genannt werden, sowie Fasern, die Cellulose, Cellulosederivate, Nylon, Polyethylen (PE), Polyamid (PA), Polyethersulfon (PES), Polyvinylidendifluorid (PVDF), Polytetrafluorethylen (PTFE), Polypropylen (PP) und Polysulfon als strukturgebenden Bestandteil umfassen, wobei diese Materialien einzeln oder in entsprechender Kombination Anwendung finden können. Vorzugsweise werden Polyesterfasern, insbesondere Fasern aus Polyethylenterephthalat oder Polybutylenterephthalat (PBT) und Polyamidfasern verwendet.

Beispielhaft für (Polymer)-Membranen, die als Material für das polymere Trägermaterial des erfindungsgemäßen Adsorptionsmediums verwendet werden können, können Membranen genannt werden, die Cellulose, Cellulosederivate, Nylon, Polyester, Polyethylen (PE), Polyamid (PA), Polyethersulfon (PES), Polyvinylidendifluorid (PVDF), Polytetrafluorethylen (PTFE), Polypropylen (PP) und Polysulfon als strukturgebenden Bestandteil umfassen, wobei diese Materialien einzeln oder in entsprechender Kombination Anwendung finden können. Vorzugsweise werden Membranen auf Basis von Cellulose und Cellulosederivate, insbesondere Cellulosehydratmembranen, oder Polyethylenmembranen verwendet.

Hierbei können in Abhängigkeit von der aufzureinigenden Lösung bekannte (Polymer)-Membranen mit unterschiedlichen Porengrößen als Ausgangmembranen verwendet werden. Beispielsweise kann erfindungsgemäß eine Celluloseester-Membran mit einer Porengröße von 0,1 bis 20 $\mu$m, vorzugsweise 0,5 bis 15 $\mu$m und mehr bevorzugt von 1 bis 10 $\mu$m als Ausgangsmembran verwendet werden, welche durch übliche, auf dem Fachgebiet bekannte Verfahren verseift und gegebenenfalls vernetzt werden kann. Die Porengröße wird üblicherweise anhand eines "Capillary Flow Porometry Test" unter Verwendung eines "Coulter Capillary Flow Porometers 6.0" und unter Verwendung des CAPWIN-Softwaresystems der Firma Porous Materials Inc. bestimmt.

Beispielhaft für Polymergele, die als Material für das polymere Trägermaterial des erfindungsgemäßen Adsorptionsmediums verwendet werden können, können Agarose, Dextran, Cellulose, Polymethacrylate, Polyvinylether, Polyacrylamide, Polystyrol-Divinylbenzol-Copolymere, Silica-Dextran, Agarose-Acrylamide und Dextran-Acrylamide genannt werden.

Beispielhaft für Folien und Gewebe können Folien und Gewebe aus den vorstehend genannten Polymermaterialien, welche für die (Polymer)-Membranen verwendet werden können, genannt werden. Beispielhaft für Vliese, die als Material für das polymere Trägermaterial des erfindungsgemäßen Adsorptionsmediums verwendet werden können, können Polyester/Polypropylen/Polyamid-Vliese (beispielsweise "Pluratexx 2317 S" der Firma Freudenberg), sowie die vorstehenden Polymermaterialien, welche für die (Polymer)-Membranen verwendet werden können, genannt werden.

Im erfindungsgemäßen Adsorptionsmedium sind vorzugsweise polymere Abstandshalterelemente an die Oberfläche des Trägermaterials gebunden, wobei die Bindung zwischen der Oberfläche des Trägermaterials und den Abstandshalterelementen vorzugsweise über die (originär vorhandenen oder durch Oberflächenmodifikation erzeugten) funktionellen Gruppen der Chromatographiematrix erfolgt bzw. erfolgt ist. Durch die polymeren Abstandshalterelemente, die im erfindungsgemäßen Adsorptionsmedium als Verbindungseinheiten zwischen der Chromatographiematrix und den multimodalen Liganden dienen, ist es vorteilhafterweise möglich, hohe Ligandendichten zu erzielen, die eine hohe

Bindungskapazität für kleine Proteine, wie beispielsweise Lysozym, und eine hohe Salztoleranz ermöglichen.

Durch die hohen Ligandendichten und die multimodalen Wechselwirkungen können auf diese Art salztolerante Adsorptionsmedien hergestellt werden, die auch bei erhöhter Salzkonzentration über hohe Proteinbindungskapazität verfügen.

Unter dem Begriff "polymere Abstandshalterelemente", auch "polymere Spacerelemente" genannt (kurz: "Abstandshalter") werden im Sinne der vorliegenden Erfindung jegliche Polymere verstanden, welche die inneren und äußeren Oberflächen der Chromatographiematrix mit den multimodalen Liganden verbinden können. Die polymeren Abstandshalterelemente der vorliegenden Erfindung unterliegen keiner besonderen Einschränkung, solange sie (bevorzugt) chemisch, aber auch physikalisch an die Oberfläche der Chromatographiematrix gebunden werden können. Allgemein können die polymeren Abstandshalterelemente aus der Gruppe, bestehend aus Polyaminen, Polyalkoholen, Polythiolen, Poly(meth)acrylaten, Poly(meth)acrylamiden, Poly-N-Alkyl(meth)acrylamiden sowie Copolymeren, bestehend aus zwei oder mehr der vorstehenden Polymere, und Copolymeren, bestehend aus einem oder mehreren der vorstehenden Polymere und aus Polymeren, die keine nucleophilen funktionellen Gruppen tragen, ausgewählt sein.

Gemäß einer bevorzugten Ausführungsform sind die polymeren Abstandshalterelemente Polyamine mit mindesten einer primären Aminogruppe, da hierdurch eine weitere Funktionalisierung der Oberfläche des polymeren Trägermaterials für die weitere Umsetzung nicht notwendig ist. Durch diese primäre Aminogruppe wird später als X-(C=O)-Bindung eine Amidbindung zu den multimodalen Liganden gebildet.

Polyamine im Sinne der vorliegenden Erfindung sind beispielsweise Polyallylamin, Polyvinylamin, Polyethylenimin (verzweigt oder linear), Poly(4-aminostyrol), Chitosan, Poly-L-lysin, Poly(N-methylvinylamin), Poly(N-methylallylamin) und Poly(oleylamine).

Dabei können alle geeigneten Polyamine verwendet werden. Bevorzugt sind jedoch Polyamine mit einer molaren Masse von mehr als 500 g/mol, insbesondere 800 bis 1.000.000 g/mol. Die polymeren Abstandshalterelemente weisen besonders bevorzugt eine molare Masse von 3.000 bis 150.000 g/mol auf, mehr bevorzugt von 10.000 bis 100.000 g/mol.

In einer besonders bevorzugten Ausführungsform sind die polymeren Abstandshalterelemente aus der Gruppe der Polyallylamine mit einer molaren Masse von 3.000 bis 150.000 g/mol, mehr bevorzugt 10.000 bis 100.000 g/mol, ausgewählt. In einer weiteren bevorzugten Ausführungsform sind die polymeren Abstandshalterelemente aus der Gruppe der Polyvinylamine mit einer molaren Masse von 5.000 bis 500.000 g/mol, mehr bevorzugt 10.000 bis 100.000 g/mol, ausgewählt.

Erfindungsgemäß sind insbesondere Polyallylamin, Polyvinylamin und/oder Polyethylenimin bevorzugt.

Im erfindungsgemäßen Adsorptionsmedium sind die polymeren Abstandshalterelemente, falls vorhanden, sowohl an die Oberfläche der Chromatographiematrix als auch an die multimodalen Liganden gebunden. Die Bindung zu den multimodalen Liganden erfolgt über eine kovalente Bindung der Gruppen -XH (entweder der Chromatographiematrix oder der Abstandshalterelemente) an die Carbonylgruppe eines Carbonsäurederivats als Ligandenvorstufe unter Ausbildung einer -X-(C=O)-Bindung mit X = -NR-, -O- oder -S- und R = Alkyl, Alkenyl, Aryl, Heteroaryl oder H. Bei einer besonders bevorzugten Ausführungsform erfolgt die Bindung zu den multimodalen Liganden über eine sekundäre Amidbindung, d.h. über eine Bindung des Typs -NH-(C=O).

Die Ligandendichte der multimodalen Liganden des erfindungsgemäßen Adsorptionsmediums beträgt vorzugsweise mindestens 25 µmol/ml, bevorzugt 100 µmol/ml, mehr bevorzugt mindestens 150 µmol/ml und besonders bevorzugt mindestens 250 µmol/ml. Die Ligandendichte wird gemäß der vorliegenden Erfindung mittels Titration bestimmt, wobei Details in der nachstehenden Methode M2 dargestellt sind.

Bei der besonders bevorzugten Ausführungsform eines Polyaminfunktionalisierten Adsorptionsmediums beträgt die Amingruppendichte, welche die Ligandendichte des Polyaminfunktionalisierten Adsorptionsmediums darstellt, bevor die multimodalen Liganden immobilisiert werden, mindestens 25 µmol/ml, vorzugsweise mindestens 150 µmol/ml, mehr bevorzugt mindestens 200 µmol/ml und besonders bevorzugt mindestens 400 µmol/ml. Die Amingruppendichte wird gemäß der vorliegenden Erfindung mittels Titration bestimmt, wobei Details in der nachstehenden Methode M1 dargestellt sind.

Gemäß der vorliegenden Erfindung weisen die multimodalen Liganden die folgende Struktur auf:

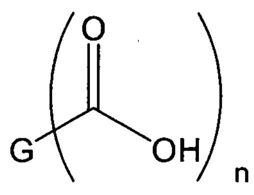,

wobei G eine verzweigte oder unverzweigte $C_{3-10}$ Alkenylgruppe darstellt, die ein oder mehrere Heteroatome, ausgewählt

aus O, S, N und Halogenen, enthalten kann und gegebenenfalls mindestens einen Hydroxyl-, Carbonyl-, Carboxyl-, Carbonsäureanhydrid- oder aromatischen Substituenten enthalten kann, und wobei n 1 ist.

Besonders bevorzugt weist der multimodale Ligand abgesehen von der Amidbindung, über welche dieser an das polymere Trägermaterial gebunden sein kann, keine weitere Amidbindung auf.

Als besonders bevorzugter Ligand kann die nachfolgende Struktur (4) genannt werden:

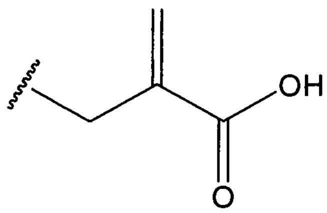

(4)

Gemäß der vorliegenden Erfindung ist ein multimodaler Ligand der folgenden Struktur nicht in den vorstehenden allgemeinen Strukturen für den Liganden -G-$(CO_2H)_n$ eingeschlossen:

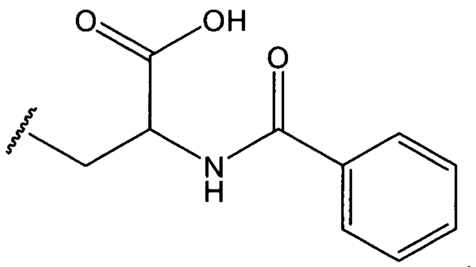

Des Weiteren stellt die vorliegende Erfindung ein Verfahren zur Herstellung des erfindungsgemäßen Adsorptionsmediums bereit. Die vorstehenden Ausführungen bezüglich des erfindungsgemäßen Adsorptionsmediums treffen daher auch auf das erfindungsgemäße Herstellungsverfahren zu.

Das erfindungsgemäße Verfahren zur Herstellung eines Adsorptionsmediums umfasst die folgenden Schritte:

(a) Bereitstellen eines polymeren Trägermaterials T, wobei das Trägermaterial T mindestens eine Gruppe -XH aufweist, welche mit Carbonsäurederivaten unter Ausbildung einer kovalenten Bindung -X-(C=O) reaktionsfähig ist, wobei X -NR-, -O- oder -S- und R = Alkyl, Alkenyl, Aryl, Heteroaryl oder Wasserstoff darstellen; und

(b) Umsetzen der mindestens einen Gruppe -XH des polymeren Trägermaterials T mit einem Carbonsäurederivat als Vorstufe eines multimodalen Liganden derart, dass die kovalente Bindung -X-(C=O) gebildet wird, über welche der multimodale Ligand an das Trägermaterial gebunden wird,

wobei der multimodale Ligand die Struktur -G-$(CO_2H)_n$ aufweist, wobei G eine verzweigte oder unverzweigte $C_{3-10}$ Alkenylgruppe darstellt, die ein oder mehrere Heteroatome, ausgewählt aus O, S, N und Halogenen, enthalten kann und gegebenenfalls mindestens einen Hydroxyl-, Carbonyl-, Carboxyl-, Carbonsäureanhydrid- oder aromatischen Substituenten enthalten kann, und wobei n 1 ist.

Bei einer besonders bevorzugten Ausführungsform des erfindungsgemäßen Verfahrens ist die kovalente Bindung -X-(C=O) eine sekundäre Amidbindung, welche durch Umsetzung eines Carbonsäureanhydrids als Vorstufe des Liganden und Amingruppen des Trägermaterials gebildet wird, und der multimodale Ligand verfügt über mindestens eine freie Carbonsäuregruppe.

Gemäß einer bevorzugten Ausführungsform, bei welcher die multimodalen Liganden über polymere Abstandshalterelemente an die Oberfläche des Trägermaterials gebunden sind, werden die polymeren Abstandshalterelemente vor dem Schritt (b) auf der Oberfläche der Chromatographiematrix immobilisiert und anschließend werden gemäß Schritt (b) die multimodalen Liganden unter Ausbildung einer -X-(C=O)-Bindung, besonders bevorzugt einer sekundären Amidbindung, an den -XH-Gruppen der Abstandshalterelemente immobilisiert.

Im Schritt (a) des erfindungsgemäßen Verfahrens wird ein wie vorstehend beschriebenes polymeres Trägermaterial bereitgestellt, das originär funktionelle Gruppen aufweist (beispielsweise Polyesterfasern) oder bei welchem durch Oberflächenmodifikation funktionelle Gruppen eingeführt wurden. Vorzugsweise werden in einem weiteren Schritt

(a*) des erfindungsgemäßen Verfahrens polymere Abstandshalterelemente auf der Oberfläche der Chromatographiematrix immobilisiert, d.h. die Abstandshalterelemente werden (bevorzugt) chemisch oder auch gegebenenfalls physikalisch an die Oberfläche der Chromatographiematrix über deren funktionelle Gruppen gebunden. Der Schritt des Immobilisierens unterliegt erfindungsgemäß keiner besonderen Einschränkung und sämtliche dem Fachmann bekannten Immobilisierungsverfahren, wie beispielsweise Substitutions- oder Additionsreaktionen, Epoxidöffnungen, Aminolysen, Amidkupplungsreaktionen, Veresterungen, reduktive Aminierungen und Insertionsreaktionen, können zur Anwendung kommen.

Im Schritt (b) des erfindungsgemäßen Verfahrens wird die mindestens eine -XH-Gruppe, die gegebenenfalls über Abstandshalterelemente auf dem Trägermaterial immobilisiert ist, mit einer Vorstufe der multimodalen Liganden derart umgesetzt, dass eine -X-(C=O)-Bindung gebildet wird, über welche die multimodalen Liganden an das Trägermaterial gebunden werden. Bei der-X-(C=O)-Bindung handelt es sich vorzugsweise um eine sekundäre Amidbindung.

Gemäß einer bevorzugten Ausführungsform der vorliegenden Erfindung wird die sekundäre Amidgruppe durch Umsetzung eines Carbonsäureanhydrids mit Aminogruppen des Trägermaterials gebildet. D.h. die Aminogruppen werden vorzugsweise mit Carbonsäureanhydriden funktionalisiert, wie dies anhand des nachfolgenden Schemas illustriert werden kann:

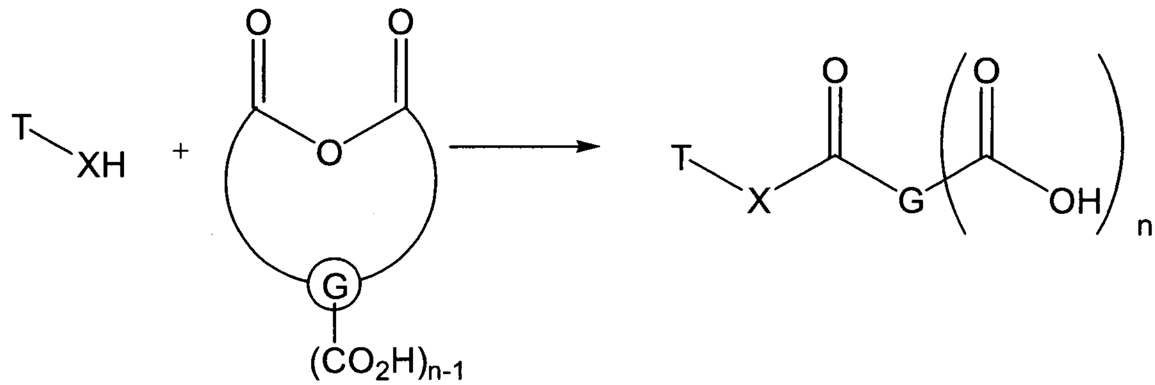

,

wobei G und n wie vorstehend definiert sind und wobei -XH = $NH_2$ ist.

Bei den Gruppen G, die selber keine Carbonsäuregruppen -COOH als Substituenten aufweisen, resultiert die einzige im Liganden $G\text{-}(CO_2H)_n$ vorhandene Carbonsäuregruppe aus der Ringöffnungsreaktion des Carbonsäureanhydrids mit der nucleophilen Gruppe -XH und n ist gleich 1.

Bei den Gruppen G, die selber mindestens eine Carbonsäuregruppe -COOH als Substituenten aufweisen, ist n nicht erfindungsgemäß größer oder gleich 2, wobei eine der Carbonsäuregruppen aus der Ringöffnungsreaktion des Carbonsäureanhydrids mit der nucleophilen Gruppe -XH resultiert.

Das Carbonsäureanhydrid unterliegt erfindungsgemäß keiner besonderen Einschränkung, solange multimodale Liganden der vorstehenden Struktur erhalten werden können. Als Carbonsäureanhydrid kann unter anderem Itaconsäureanhydrid eingesetzt werden.

Als Lösungsmittel können Dimethylsulfoxid, 2-Pyrrolidon, Dimethylformamid, Dimethylacetamid, Tetrahydrofuran oder 1,4-Dioxan oder andere polare, bevorzugter Weise aprotische Lösungsmittel eingesetzt werden.

Des Weiteren stellt die vorliegende Erfindung die Verwendung des erfindungsgemäßen Adsorptionsmediums oder eines gemäß dem erfindungsgemäßen Verfahren hergestellten Adsorptionsmediums zur Aufreinigung von Biomolekülen bereit. Bei den aufzureinigenden Biomolekülen handelt es sich erfindungsgemäß um Proteine, wie z.B. Antikörper, Peptide, Aminosäuren, Nukleinsäuren, Virus-artige Partikel, Viren und/oder Endotoxine.

Durch die polymeren Abstandshalterelemente, die im erfindungsgemäßen Adsorptionsmedium als Verbindungseinheiten zwischen der Chromatographiematrix und den multimodalen Liganden dienen, ist es vorteilhafterweise möglich, hohe Ligandendichten zu erzielen, die eine hohe Bindungskapazität insbesondere für kleine Proteine, wie beispielsweise Lysozym, ermöglichen. Durch die hohen Ligandendichten und die multimodalen Wechselwirkungen können durch das erfindungsgemäße Verfahren salztolerante Adsorptionsmedien hergestellt werden, die auch bei erhöhter Salzkonzentration von bis zu 500 mM (NaCl-Konzentration) über hohe Proteinbindungskapazität verfügen. Durch Wahl des Anhydrids lässt sich das Maximum der Bindungskapazität der Kationenaustausch-Adsorptionsmedien über einen weiten Salzkonzentrationsbereich gezielt einstellen. Da die multimodale Ligandenstruktur mit Hilfe von nur einem Reagenz, beispielsweise einem Carbonsäureanhydrid, erzeugt wird, ist auf dem Adsorptionsmedium das Verhältnis von Carbonsäuregruppen zu den Gruppen, die weitere Wechselwirkungen ermöglichen, stets konstant. Dadurch ist das Ergebnis des Herstellungsprozesses im Vergleich zu einem Modifizierungsverfahren mit zwei unterschiedlichen Liganden, die jeweils separat auf dem Adsorptionsmedium fixiert werden, oder unvollständigen Umsetzungen stets reproduzierbar. Das erfindungsgemäße Adsorptionsmedium eignet sich daher hervorragend zur Aufreinigung von Biomolekülen, woran ein großes industrielles Interesse besteht.

Die vorliegende Erfindung wird anhand der folgenden, nicht einschränkenden Beispiele näher erläutert, wobei die in den Figuren 1 bis 3 dargestellten Diagramme die Bindungskapazitäten der erhaltenen Membranen zusammenfassen.

## Beispiele

Methoden:

*M1: Bestimmung der Liganden-/Ladungsdichte von Amin-funktionalisierten Adsorptionsmedien*

Drei Lagen Membran wurden in einen Membranhalter eingespannt. Der Membranstapel wies eine Membranfläche von 15 $cm^2$, eine Anströmfläche von 5 $cm^2$ und eine Betthöhe (Dicke des Membranstapels) von 750 $\mu$m in dem Membranhalter auf. Die Membranen in dem Membranhalter wurden mit 20 mM TRIS/HCl Puffer bei pH = 7,4 geflutet, um die Luft zu verdrängen, und dann an eine FPLC-Anlage Äkta Explorer 100 der Firma General Electric Health Care angeschlossen. Danach wurden die Membranen bzw. der Membranstapel mit einem vier Schritte umfassenden Testprogramm hinsichtlich der Ladungsdichte untersucht. Die vier Schritte des Testprogramms sind nachfolgend angegeben:

1. Konditionieren der Membran mit 6 mL 1 M NaCl-Lösung in 20 mM TRIS/HCl bei pH = 7.4
2. Regenerieren der Membran mit 6 mL einer 1 M Lösung von NaOH in RO-Wasser
3. Waschen der Membran mit 100 mL RO-Wasser und
4. Beladen der Membran mit 135 mL 10 mM HCl

Alle Schritte wurden bei einer Fließgeschwindigkeit von 10 mL/min durchgeführt. Bei allen Schritten wurde die Leitfähigkeit im Detektor hinter der Membraneinheit gemessen. Die Fläche oberhalb der so aufgezeichneten Durchbruchskurve wurde nach dem Abziehen des Totvolumens integriert und daraus die Ladungsdichte berechnet.

*M2: Bestimmung der Liganden-/Ladungsdichte von Kationenaustausch-Adsorptionsmedien*

Drei Lagen Membran wurden in einen Membranhalter eingespannt. Der Membranstapel wies eine Membranfläche von 15 $cm^2$, eine Anströmfläche von 5 $cm^2$ und eine Betthöhe (Dicke des Membranstapels) von 750 $\mu$m in dem Membranhalter auf. Die Membranen in dem Membranhalter wurden mit 20 mM KPi-Puffer bei pH = 7 geflutet, um die Luft zu verdrängen, und dann an eine FPLC-Anlage Äkta Explorer 100 der Firma General Electric Health Care angeschlossen. Danach wurden die Membranen bzw. der Membranstapel mit einem vier Schritte umfassenden Testprogramm hinsichtlich der Ladungsdichte untersucht. Die vier Schritte des Testprogramms sind nachfolgend angegeben:

1. Konditionieren der Membran mit 6 mL 1 M NaCl-Lösung in 20 mM KPi bei pH = 7,0
2. Regenerieren der Membran mit 6 mL einer 1 M-Lösung von HCl in RO-Wasser
3. Waschen der Membran mit 88 mL RO-Wasser und
4. Beladen der Membran mit 135 mL 10 mM NaOH

Alle Schritte wurden bei einer Fließgeschwindigkeit von 10 mL/min durchgeführt. Bei allen Schritten wurde die Leitfähigkeit im Detektor hinter der Membraneinheit gemessen. Die Fläche oberhalb der so aufgezeichneten Durchbruchskurve wurde nach dem Abziehen des Totvolumens integriert und daraus die Ladungsdichte berechnet.

*M3: Bestimmung der Bindungskapazität für Lysozym von modifizierten Membranen mittels Durchbruchskurve*

Drei Lagen Membran wurden in einen Membranhalter eingespannt. Der Membranstapel wies eine Membranfläche von 15 $cm^2$, eine Anströmfläche von 5 $cm^2$ und eine Betthöhe (Dicke des Membranstapels) von 900 $\mu$m in dem Membranhalter auf. Die Membranen in dem Membranhalter wurden mit 10 mM KPi-Puffer bei pH = 7 geflutet, um die Luft zu verdrängen, und dann an eine FPLC-Anlage Äkta Explorer 100 der Firma General Electric Health Care angeschlossen. Danach wurden die Membranen bzw. der Membranstapel mit einem drei Schritte umfassenden Testprogramm hinsichtlich der Lysozym-Bindungskapazität untersucht. Die drei Schritte des Testprogramms sind nachfolgend angegeben:

1. Konditionieren der Membran mit 20 mL 1 M NaCl-Lösung in 10 mM KPi bei pH = 7,0
2. Equilibrieren der Membran mit 20 mL Bindungspuffer (10 mM KPi, pH = 7,0)

3. Beladen der Membran mit 250 mL Lysozym-Lösung (0,20 % Lysozym in Bindungspuffer)

Alle Schritte wurden bei einer Fließgeschwindigkeit von 10 mL/min durchgeführt. Bei allen Schritten wurde die Absorption bei 280 nm im Detektor hinter der Membraneinheit gemessen. Die Fläche oberhalb der so aufgezeichneten Durchbruchskurve wurde nach dem Abziehen des Totvolumens integriert und daraus die Menge an gebundenem Lysozym berechnet.

*M4: Bestimmung der Bindungskapazität für y-Globulin von modifizierten Membranen mittels Durchbruchskurve*

Drei Lagen Membran wurden in einen Membranhalter eingespannt. Der Membranstapel wies eine Membranfläche von 15 $cm^2$, eine Anströmfläche von 5 $cm^2$ und eine Betthöhe (Dicke des Membranstapels) von 900 μm in dem Membranhalter auf. Die Membranen in dem Membranhalter wurden mit 20 mM NaAc-Lösung bei pH = 5 geflutet, um die Luft zu verdrängen, und dann an eine FPLC-Anlage Äkta Explorer 100 der Firma General Electric Health Care angeschlossen. Danach wurden die Membranen bzw. der Membranstapel mit einem drei Schritte umfassenden Testprogramm hinsichtlich der y-Globulin-Bindungskapazität untersucht. Die drei Schritte des Testprogramms sind nachfolgend angegeben:

1. Konditionieren der Membran mit 20 mL 1 M NaCl-Lösung in 20 mM NaAc bei pH = 5,0
2. Equilibrieren der Membran mit 20 mL Bindungspuffer (25 mM NaCl in 20 mM NaAc bei pH = 5,0)
3. Beladen der Membran mit 250 mL 1mg/mL γ-Globulin-Lösung in Bindungspuffer

Alle Schritte wurden bei einer Fließgeschwindigkeit von 10 mL/min durchgeführt. Bei allen Schritten wurde die Absorption bei 280 nm im Detektor hinter der Membraneinheit gemessen. Die Fläche oberhalb der so aufgezeichneten Durchbruchskurve wurde nach dem Abziehen des Totvolumens integriert und daraus die Menge an gebundenem γ-Globulin berechnet. Die Messung wurde mit einer frischen Membranprobe mit 150 mM NaCl und 300 mM NaCl wiederholt.

**Modifizierungsprotokoll für die Immobilisierung von Carbonsäureanhydriden auf aminmodifizierten Ausgangsmatrizen**

**Modifizierung von Cellulosehydratmembranen**

1. Polyaminimmobilisierung

1a) Polyallylamin (PAA)

Die Abstandshalterimmobilisierung ist angelehnt an ein bekanntes Protokoll, welches in DE 10 2008 055 821 A1 (Beispiele 21 und 22) beschrieben wurde. Hierbei werden Abstandshalter mit einer molaren Masse von 15000 g/mol bis 150000 g/mol verwendet. In einer typischen Umsetzung wurde die Celluloseacetat-(CA)-Membran (3 μm Porengröße, Sartorius Stedim Biotech GmbH) in einer 0,6 M wässrigen Natriumhydroxidlösung (4 g/$cm^2$) für 30 min bei Raumtemperatur verseift und anschließend dreimal für 10 min in einer 0,25 M Natriumhydroxidlösung (0,5 g/$cm^2$) gespült. Die erhaltene Membran wurde für 30 min mit einer Lösung, bestehend aus 15 % 1,4-Butandioldiglycidylether und 85 % 0,25 M wässriger Natriumhydroxidlösung (0,5 g/$cm^2$) behandelt und anschließend für 18 h in einem verschlossenen Gefäß bei Raumtemperatur gelagert. Abschließend wurde 30 min mit fließendem Wasser gespült.

Die so erhaltene Membran wurde für 1 h mit einer 20 %igen Lösung von Polyallylamin in RO-Wasser (1 g/$cm^2$) bei 50 °C behandelt. Die Membran wurde anschließend 5 min bei Raumtemperatur mit 5 %iger Schwefelsäurelösung behandelt und abschließend für 10 min mit fließendem Wasser gespült.

Die Amingruppendichte auf der Membran wurde über Titration bestimmt.

| Trägermaterial | Amingruppendichte |
|---|---|
| PAA-modifizierte Cellulosehydratmembranen | 450-550 μmol/mL |

1b) Polyethylenimin (PEI)

Die Abstandshalterimmobilisierung ist angelehnt an ein bekanntes Protokoll, welches in DE 10 2008 055 821 A1 (Beispiele 15, 16 und 17) beschrieben wurde. In einer typischen Umsetzung wurde die CA-Membran (3 μm Porengröße, Sartorius Stedim Biotech GmbH) in einer 0,6 M wässrigen Natriumhydroxidlösung (4 g/$cm^2$) für 30 min bei

Raumtemperatur verseift und anschließend dreimal für 10 min in einer 0,25 M Natriumhydroxidlösung (0,5 g/cm²) gespült. Die erhaltene Membran wurde für 30 min mit einer Lösung, bestehend aus 15 % 1,4-Butandioldiglycidylether und 85 % 0,25 M wässriger Natriumhydroxidlösung (0,5 g/cm²) behandelt und anschließend für 18 h in einem verschlossenen Gefäß bei Raumtemperatur gelagert. Abschließend wurde 30 min mit fließendem Wasser gespült. Die so erhaltene Membran wurde für 2 h mit einer 30 %igen Lösung von Lupasol WF (Polyethylenimin der BASF AG, Molmasse 25000 g/mol) in RO-Wasser (1 g/cm²) bei 50 °C behandelt. Die Membran wurde anschließend 30 min mit fließendem Wasser gespült, 10 min mit 5 %iger Schwefelsäurelösung behandelt und abschließend für 10 min mit fließendem Wasser gespült.

Die Amingruppendichte auf der Membran wurde über Titration bestimmt.

| **Trägermaterial** | **Amingruppendichte** |
|---|---|
| PEImodifizierte Cellulosehydratmembranen | 600-650 pmol/mL |

2. Ligandenimmobilisierung

In einer typischen Umsetzung wurde 16 g Carbonsäureanhydrid in 64 g DMSO (20 Gew.%) gelöst und auf 60 °C erwärmt. Die PAA-modifizierte Cellulosehydratmembran wurde in die Reaktionslösung (0,5 g/cm²) gelegt und bei 60 °C für 1 h geschüttelt. Anschließend wurde die Reaktionslösung abfiltriert und die Membran mit Ethanol (0,5 g/cm²) und einem großen Überschuss an RO-Wasser gewaschen.

Ligandenstrukturen:

Die hier aufgeführten Kationentauscher wurden nach der oben beschriebenen Methode hergestellt, wobei folgende Carbonsäureanhydride verwendet wurden. Die Ergebnisse sind in den folgenden Tabellen 1 bis 3 und in den Figuren 1 bis 3 gezeigt.

| **Beispiel** | **Anhydrid** |
|---|---|
| 1 | Bernsteinsäureanhydrid |
| 2 | Glutarsäureanhydrid |
| 3 | Äpfelsäureanhydrid |
| 4 | Itaconsäureanhydrid |
| 5 | Maleinsäureanhydrid |
| 6 | Phthalsäureanhydrid |
| 7 | Chinolinsäureanhydrid |
| 8 | Trimellitsäureanhydrid |
| 9 | Pyromellitsäureanhydrid |
| 10 | 4-((2,5-Dioxotetrahydrofuran-3-yl)thio)benzoesäure |
| 11 | *N*-(2,5-Dioxotetrahydrofuran-3-yl)acetamid |
| 12 | *N*-(2,5-Dioxotetrahydrofuran-3-yl)-2,2,2-trifluoracetamid |
| 13 | Maleinsäureanhydrid |
| 14 | Maleinsäureanhydrid |
| 15 | Bernsteinsäureanhydrid |
| 16 | Maleinsäureanhydrid |
| V-1* | N-Benzoyl-L-Asparaginsäureanhydrid |
| *) vergleichsbeispiel 1 | |

Beispiele 1-3 und 5-16 sind nicht erfindungsgemäß.

Im Vergleich hierzu wurde zudem ein im Stand der Technik bekannter starker Kationenaustauscher-Membranadsorber Sartobind S (starker Kationenaustauscher aus Cellulosehydrat mit Sulfonsäureliganden, Sartorius Stedim Biotech GmbH) getestet. Die Ergebnisse sind in der folgenden Tabelle 4 sowie und in den Figuren 1 bis 3 als "Ref" gezeigt.

Darüber hinaus wurde die Umsetzung von N-Benzoyl-L-Asparaginsäureanhydrid mit der PAA-modifizierten Cellulosehydratmembran (molare Masse von PAA: 15.000 g/mol) als Vergleichsbeispiel 1 durchgeführt, wodurch eine Chromatographiematrix mit einem 2-Benzoylamino-butansäureligand erhalten wurde, welcher den im Stand der Technik bekannten Capto™ MMC Liganden nachstellen soll.

Tabelle 1: Abstandshalter Polyallylamin (M = 15.000 g/mol)

| | **Polyamin** | **Struktur** | **Kapazität Lysozym [mg/mL]M3** | **Kapazität Globulin 25 mM NaCl [mg/mL]M4** | **Kapazität Globulin 150 mM NaCl [mg/mL]M4** | **Kapazität Globulin 300 mM NaCl [mg/mL] M4** | **Ligandendichte Polyaminfunktionalisierte Membran [µmol/mL] M1** | **Ligandendichte Anhydridfunktionalisierte Membran [µmol/mL] M2** |
|---|---|---|---|---|---|---|---|---|
| **1** | PAA (15.000 g/mol) | 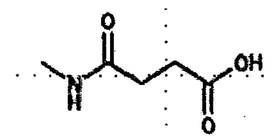 | 137,1 | 60,0 | 20,0 | 0,3 | 494 | 431 |
| **2** | PAA (15.000 g/mol) | 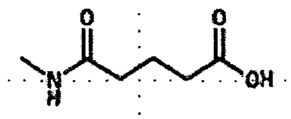 | 191,7 | 97,2 | 30,6 | 0,8 | 494 | 438 |
| **3** | PAA (15.000 g/mol) | 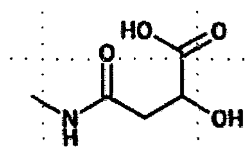 | 87,5 | 31,3 | 21,9 | 9,4 | 494 | 290 |
| **4** | PAA (15.000 g/mol) | 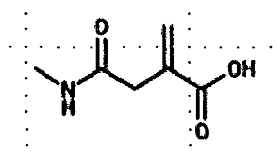 | 94,3 | 22,9 | 31,4 | 14,3 | 550 | 574 |
| **5** | PAA (15.000 g/mol) | 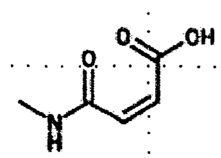 | 123,5 | 23,5 | 42,4 | 23,5 | 494 | 441 |
| **6** | PAA (15.000 g/mol) | 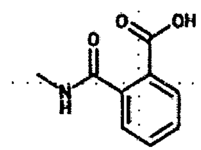 | 123,7 | 15,0 | 15,7 | 22,0 | 524 | 525 |

(fortgesetzt)

| | Polyamin | Struktur | Kapazität Lysozym [mg/mL] M3 | Kapazität Globulin 25 mM NaCl [mg/mL] M4 | Kapazität Globulin 150 mM NaCl [mg/mL] M4 | Kapazität Globulin 300 mM NaCl [mg/mL] M4 | Ligandendichte Polyaminfunktionalisierte Membran [μmol/mL] M1 | Ligandendichte Anhydridfunktionalisierte Membran [μmol/mL]M2 |
|---|---|---|---|---|---|---|---|---|
| **7** | PAA (15.000 g/mol) | 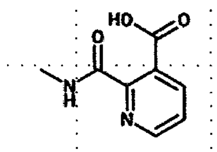 | 81,2 | 29,7 | 37,1 | 19,4 | 494 | 425 |
| **8** | PAA (15.000 g/mol) | 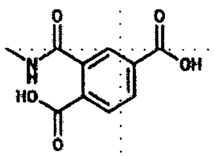 | 61,1 | 16,0 | 24,5 | 25,3 | 494 | 760 |
| **9** | PAA (15.000 g/mol) | 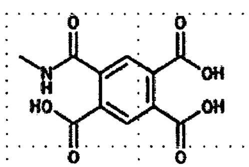 | 83,3 | 16,7 | 30,6 | 22,2 | 494 | 833 |
| **10** | PAA (15.000 g/mol) | 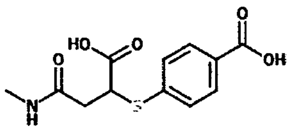 | 76,8 | 13,0 | 13,3 | 18,3 | 546 | 640 |
| **11** | PAA (15.000 g/mol) | 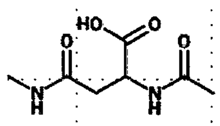 | 111,4 | 57,1 | 14,3 | 16,9 | 546 | 437 |
| **12** | PAA (15.000 g/mol) | 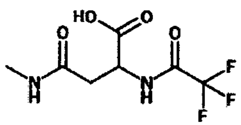 | 83,4 | 39,1 | 40,7 | 5,4 | 546 | 534 |

(fortgesetzt)

| | Polyamin | Struktur | Kapazität Lysozym [mg/mL] M3 | Kapazität Globulin 25mM NaCl [mg/mL]M4 | Kapazität Globulin 150 mM NaCl [mg/mL] M4 | Kapazität Globulin 300 mM NaCl [mg/mL] M4 | Ligandendichte Polyaminfunktionalisierte Membran [μmol/mL] M1 | Ligandendichte Anhydridfunktionalisierte Membran [μmol/mL] M2 |
|---|---|---|---|---|---|---|---|---|
| **V-1*** | PAA (15.000 g/mol) | 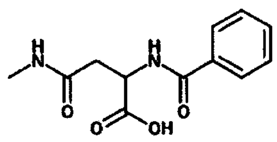 | 55,3 | 15,3 | 17,1 | 24,0 | 494 | 473 |

*) Vergleichsbeispiel 1

Tabelle 2: Abstandshalter Polyallylamin (M = 100.000 g/mol und 150.000 g/mol)

| | Polyamin | Struktur | Kapazität Lysozym [mg/mL] M3 | Kapazität Globulin 25 mM NaCl [mg/mL] M4 | Kapazität Globulin 150 mM NaCl [mg/mL] M4 | Kapazität Globulin 300 mM NaCl [mg/mL] M4 | Ligandendichte Polyaminfunktionalisierte Membran [μmol/mL] M1 | Ligandendichte Anhydridfunktionalisierte Membran [μmol/mL] M2 |
|---|---|---|---|---|---|---|---|---|
| **13** | PAA (150.000 g/mol) | 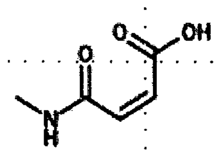 | 150,0 | 26,7 | 56,7 | 33,3 | 843 | 506 |
| **14** | PAA (100.000 g/mol) | 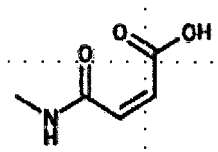 | 120,0 | 31,4 | 54,3 | 25,7 | 667 | 345 |

Tabelle 3: Abstandshalter Polyethylenimin (M = 25.000g/mol)

| | Polyamin | Struktur | Kapazität Lysozym [mg/mL] M3 | Kapazität Globulin 25 mM NaCl [mg/mL] M4 | Kapazität Globulin 150 mM NaCl [mg/mL] M4 | Kapazität Globulin 300 mM NaCl [mg/mL] M4 | Ligandendichte Polyethyleniminfunktionalisierte Membran [µmol/mL] M1 | Ligandendichte Anhydridfunktionalisierte Membran [µmol/mL] M2 |
|---|---|---|---|---|---|---|---|---|
| **15** | PEI (25.000 g/mol) | 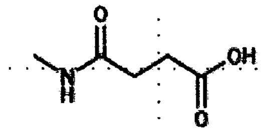 | 90,7 | 6,7 | 3,3 | 1,1 | 650 | 373 |
| **16** | PEI (25.000 g/mol) | 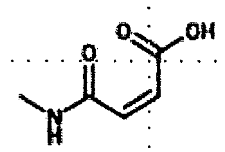 | 61,9 | 35,5 | 9,7 | 2,1 | 650 | 548 |

Tabelle 4: Membranadsorber Sartobind S (starker Kationenaustauscher aus Cellulosehydrat mit Sulfonsäureliganden, Sartorius Stedim Biotech GmbH)

| | Struktur | Kapazität Lysozym [mg/mL] M3 | Kapazität Globulin 25 mM NaCl [mg/mL] M4 | Kapazität Globulin 150 mM NaCl [mg/mL] M4 | Kapazität Globulin 300 mM NaCl [mg/mL] M4 | Ligandendichte [µmol/mL] M2 |
|---|---|---|---|---|---|---|
| Ref | Sartobind S | 46 | 37 | 14 | 2 | 96 |

**Modifizierung von Polyethylenmembranen**

1. Ligandenimmobilisierung

Als Ausgangsmaterial für die Ligandenimmobilisierung wurden Polyallylamin-funktionalisierte Polyethylenmembranen Chromasorb (0,65 µm Porengröße, EMD Millipore) verwendet. In einer typischen Umsetzung wurden 16 g Carbonsäureanhydrid in 64 g DMSO (20 Gew.%) gelöst und auf 60 °C erwärmt. Die Polyallylamin-funktionalisierte Polyethylenmembran wurde in die Reaktionslösung (0,5 g/cm$^2$) gelegt und bei 60 °C für 1 h geschüttelt. Anschließend wurde die Reaktionslösung abfiltriert und die Membran mit Ethanol (0,5 g/cm$^2$) und einem großen Überschuss an RO-Wasser gewaschen.

Die hier aufgeführten Kationentauscher wurden nach der oben beschriebenen Methode hergestellt, wobei folgende Carbonsäureanhydride verwendet wurden. Die Ergebnisse sind in der folgenden Tabelle 5 gezeigt.

| Beispiel | Anhydrid |
|---|---|
| 17 | Bernsteinsäureanhydrid |
| 18 | Maleinsäureanhydrid |

Beispiele 17 und 18 sind nicht erfindungsgemäß.

Tabelle 5:

| | Struktur | Kapazität Lysozym [mg/mL] M3 | Kapazität Globulin 25 mM NaCl [mg/mL] M4 | Kapazität Globulin 150 mM NaCl [mg/mL] M4 | Kapazität Globulin 300 mM NaCl [mg/mL] M4 | Ligandendichte Polyaminfunktionalisierte Membran [µmol/mL] M1 | Ligandendichte Anhydridfunktionalisierte Membran [µmol/mL] M2 |
|---|---|---|---|---|---|---|---|
| 17 | 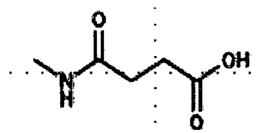 | 195,8 | 132,5 | 58,3 | 3,3 | 858 | 533 |
| 18 | 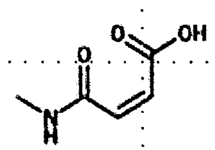 | 141,7 | 47,5 | 71,7 | 33,3 | 858 | 500 |

**Auswertung der Ergebnisse**

Die Ergebnisse sind in den Figuren 1 bis 3 zusammengefasst. Wie in Figur 1 dargestellt, zeigen im Vergleich zu der im Vergleichsbeispiel 1 erhaltenen Membran die erfindungsgemäße Membran mit den multimodalen Liganden bei vergleichbarer Ligandendichte überraschenderweise eine deutlich höhere Bindungskapazität zu kleinen Molekülen, wie z.B. Lysozym. Gleiches gilt für den im Stand der Technik bekannten starken Kationenaustauscher-Membranadsorber Sartobind S.

Zudem zeigen alle Ausführungsbeispiele gute Bindungseigenschaften für größere Moleküle, wie Globulin, über einen breiten Salzbereich (25 mM bis 300 mM NaCl). Um diese Bindungskapazität besser zu beschreiben wird eine mittlere Kapazität für Globulin $\overline{BC}$ (*Globulin*) definiert:

$$\overline{BC}\,(Globulin) = \frac{BC(Globulin{,}25\ mM\ NaCl) + BC(Globulin{,}150\ mM\ NaCl) + BC(Globulin{,}300\ mM\ NaCl)}{3}$$

Die Ergebnisse sind in Figur 2 zusammengefasst.

Um eine Aussage über die Leistungsfähigkeit der einzelnen Ausführungsbeispiele für eine Vielzahl von Anwendungen treffen zu können, wird nachfolgend die Bindungskapazität für kleine Moleküle (Lysozym) wie auch große Moleküle (Globulin) berücksichtigt. Hierfür wird ein Bindungsindikator $\overline{BC}$ (*gesamt*) definiert:

$$\overline{BC}\,(gesamt) = \frac{BC(Lysozym) + \overline{BC}\,(Globulin)}{2}$$

Die Ergebnisse sind in Figur 3 zusammengefasst. Überraschenderweise zeigt die erfindungsgemäße Membran einen deutlich erhöhten Bindungsindikator $\overline{BC}$ (*gesamt*) im Vergleich zu der im Vergleichsbeispiel 1 erhaltenen Membran. Gleiches gilt für den im Stand der Technik bekannten starken Kationenaustauscher-Membranadsorber Sartobind S.

## Patentansprüche

1. Multimodales Adsorptionsmedium, umfassend ein polymeres Trägermaterial T, an welches multimodale Liganden der folgenden Struktur -G-$(CO_2H)_n$ über eine Gruppe -X-(C=O) kovalent gebunden sind

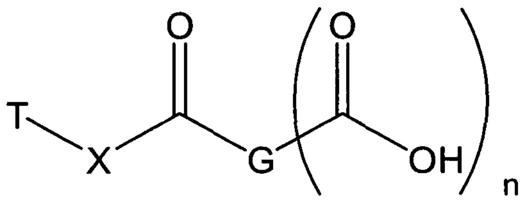

,

wobei X -NR-, -O- oder-S- und R Alkyl, Alkenyl, Aryl, Heteroaryl oder Wasserstoff darstellen,
wobei G eine verzweigte oder unverzweigte $C_{3-10}$ Alkenylgruppe darstellt, die ein oder mehrere Heteroatome, ausgewählt aus O, S, N und Halogenen, enthalten kann und gegebenenfalls mindestens einen Hydroxyl-, Carbonyl-, Carboxyl-, Carbonsäureanhydrid- oder aromatischen Substituenten enthalten kann, und wobei n 1 ist.

2. Multimodales Adsorptionsmedium nach Anspruch 1, wobei die Gruppe -X-(C=O) gleich -NH-(C=O) ist.

3. Multimodales Adsorptionsmedium nach Anspruch 1 oder 2, wobei das polymere Trägermaterial T mindestens ein Material umfasst, ausgewählt aus der Gruppe, bestehend aus natürlichen oder synthetischen Fasern, (Polymer-)Membranen, porösen, polymeren monolithischen Formkörpern, Polymergelen, Folien, Vliesen und Geweben.

4. Multimodales Adsorptionsmedium nach einem der Ansprüche 1 bis 3, wobei die multimodalen Liganden über polymere Abstandshalterelemente an die Oberfläche des Trägermaterials T gebunden sind.

5. Multimodales Adsorptionsmedium nach Anspruch 4, wobei die polymeren Abstandshalterelemente Polyamine mit mindesten einer primären Aminogruppe sind, welche als X-(C=O)-Bindung eine Amidbindung zu den multimodalen

Liganden bildet.

6. Verfahren zur Herstellung eines Adsorptionsmediums nach einem der Ansprüche 1 bis 5, umfassend die folgenden Schritte:

(a) Bereitstellen eines polymeren Trägermaterials T, wobei das Trägermaterial T mindestens eine Gruppe -XH aufweist, welche mit Carbonsäurederivaten unter Ausbildung einer kovalenten Bindung -X-(C=O) reaktionsfähig ist, wobei X -NR-, -O- oder-S- und R Alkyl, Alkenyl, Aryl, Heteroaryl oder Wasserstoff darstellen; und
(b) Umsetzen der mindestens einen Gruppe -XH des polymeren Trägermaterials T mit einem Carbonsäurederivat als Vorstufe eines multimodalen Liganden derart, dass die kovalente Bindung -X-(C=O) gebildet wird, über welche der multimodale Ligand an das Trägermaterial T gebunden wird,
wobei der multimodale Ligand die Struktur $-G-(CO_2H)_n$ aufweist,
wobei G eine verzweigte oder unverzweigte $C_{3-10}$ Alkenylgruppe darstellt, die ein oder mehrere Heteroatome, ausgewählt aus O, S, N und Halogenen, enthalten kann und gegebenenfalls mindestens einen Hydroxyl-, Carbonyl-, Carboxyl-, Carbonsäureanhydrid- oder aromatischen Substituenten enthalten kann, und wobei n 1 ist.

7. Verfahren nach Anspruch 6, wobei die kovalente Bindung -X-(C=O) eine sekundäre Amidbindung ist, welche durch Umsetzung eines Carbonsäureanhydrids als Vorstufe des Liganden und Amingruppen des Trägermaterials T gebildet wird, und wobei der multimodale Ligand über mindestens eine freie Carbonsäuregruppe verfügt.

8. Verwendung des multimodalen Adsorptionsmediums nach einem der Ansprüche 1 bis 5 zur Aufreinigung von Biomolekülen, wobei die Biomoleküle Proteine, Peptide, Aminosäuren, Nukleinsäuren, Viren, Virus-artige Partikel und/oder Endotoxine sind.

9. Verwendung nach Anspruch 8, wobei die Proteine Antikörper sind.

## Claims

1. A multimodal adsorption medium, comprising a polymeric carrier material T to which multimodal ligands of the following structure $-G-(CO_2H)_n$ are covalently bonded via an -X-(C=O) group,

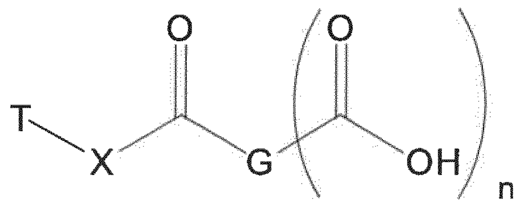

,

wherein X represents -NR-, -O- or -S- and R represents alkyl, alkenyl, aryl, heteroaryl or hydrogen,
wherein G represents a branched or unbranched $C_{3-10}$ alkenyl group which may contain one or more heteroatoms selected from O, S, N and halogens, and which may optionally contain at least one hydroxyl, carbonyl, carboxyl, carboxylic anhydride or aromatic substituent, and
wherein n is 1.

2. The multimodal adsorption medium according to claim 1, wherein the -X-(C=O) group is -NH-(C=O).

3. The multimodal adsorption medium according to claim 1 or 2, wherein the polymeric carrier material T comprises at least one material selected from the group consisting of natural or synthetic fibers, (polymer) membranes, porous polymeric monolithic molded bodies, polymer gels, films, nonwovens and wovens.

4. The multimodal adsorption medium according to any one of claims 1 to 3, wherein the multimodal ligands are bound to the surface of the carrier material T via polymeric spacer elements.

5. The multimodal adsorption medium according to claim 4, wherein the polymeric spacer elements are polyamines having at least one primary amino group, which as an X-(C=O) bond forms an amide bond with the multimodal ligands.

**6.** The method for producing an adsorption medium according to any one of claims 1 to 5, comprising the following steps:

(a) providing a polymeric carrier material T, wherein the carrier material T has at least one -XH group which is reactive with carboxylic acid derivatives while forming a covalent bond -X-(C=O), wherein X represents -NR-, -O- or -Sand R represents alkyl, alkenyl, aryl, heteroaryl or hydrogen; and
(b) reacting the at least one -XH group of the polymeric carrier material T with a carboxylic acid derivative as a precursor of a multimodal ligand such that the covalent bond -X-(C=O) is formed via which the multimodal ligand is bonded to the carrier material T,
wherein the multimodal ligand has the structure $-G-(CO_2H)_n$,
wherein G represents a branched or unbranched $C_{3-10}$ alkenyl group which may contain one or more heteroatoms selected from O, S, N and halogens, and which may optionally contain at least one hydroxyl, carbonyl, carboxyl, carboxylic anhydride or aromatic substituent, and
wherein n is 1.

**7.** The method according to claim 6, wherein the covalent bond -X-(C=O) is a secondary amide bond which is formed by reacting a carboxylic anhydride as a precursor of the ligand and amine groups of the carrier material T, and wherein the multimodal ligand has at least one free carboxylic acid group.

**8.** Use of the multimodal adsorption medium according to any one of claims 1 to 5 for the purification of biomolecules, wherein the biomolecules are proteins, peptides, amino acids, nucleic acids, viruses, virus-like particles and/or endotoxins.

**9.** Use according to claim 8, wherein the proteins are antibodies.

**Revendications**

**1.** Milieu d'adsorption multimodal, comprenant une matière de support T polymère, à laquelle des ligands multimodaux de la structure suivante $-G(CO_2H)_n$ sont liés par covalence par un groupement -X-(C=O)

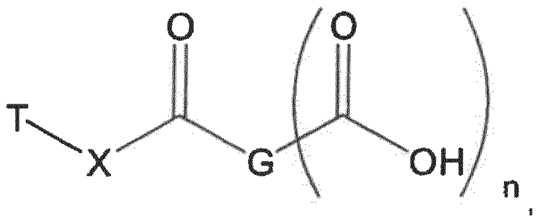

Dans laquelle X représente -NR-, -O- ou -S- et R représentant un alkyle, un alcényle, un aryle, un hétéroaryle ou l'hydrogène,
G représente un groupement alcényle en $C_3$-$C_{10}$ ramifié ou non ramifié qui peut contenir un ou plusieurs hétéroatomes sélectionnés parmi O, S, N et des halogènes et peut éventuellement contenir au moins un substituant hydroxyle, carbonyle, carboxyle, anhydride d'acide carboxylique ou aromatique et n étant 1.

**2.** Milieu d'adsorption multimodal selon la revendication 1, le groupement -X-(C=O) étant égal à -NH-(C=O).

**3.** Milieu d'adsorption multimodal selon la revendication 1 ou 2, la matière de support T polymère comprenant au moins une matière sélectionnée parmi le groupe consistant en fibres naturelles ou fibres synthétiques, membranes (polymères), des corps moulés monolithique poreux, polymères, des gels polymères, des feuilles, des non-tissés et des tissus.

**4.** Milieu d'adsorption multimodal selon l'une des revendications 1 à 3, les ligands multimodaux étant liés par des éléments d'entretoise polymères à la surface de la matière de support T.

**5.** Milieu d'adsorption multimodal selon la revendication 4, les éléments d'entretoise polymères étant des polyamines avec au moins un groupement amino primaire, qui forme comme liaison X-(C=O) une liaison amide aux ligands multimodaux.

**6.** Procédé de fabrication d'un milieu d'adsorption selon l'une des revendications 1 à 5, comprenant les étapes suivantes:

(a) fourniture d'une matière de support T polymère, la matière de support T présentant au moins un groupement -XH, lequel peut réagir avec des dérivés d'acide carboxylique avec formation d'une liaison covalente -X-(C=O), X représentant -NR-, - O- ou -S- et R représentant un alkyle, un alcényle, un aryle, un hétéroaryle ou l'hydrogène; et
(b) réaction dudit au moins un groupement -XH de la matière de support T polymère avec un dérivé d'acide carboxylique comme étape préalable d'un ligand multimodal de sorte que la liaison covalente -X-(C=O) est formée, par laquelle le ligand multimodal est lié à la matière de support T,
le ligand multimodal présentant la structure $-G-(CO_2H)_n$,
G représentant un groupement alcényle en $C_3$-$C_{10}$ ramifié ou non ramifié qui peut contenir un ou plusieurs hétéroatomes sélectionnés parmi O, S, N et des halogènes et peut éventuellement contenir au moins un substituant hydroxyle, carbonyle, carboxyle, anhydride d'acide carboxylique ou aromatique, et n étant 1.

**7.** Procédé selon la revendication 6, la liaison covalente -X-(C=O) étant une liaison amide secondaire, laquelle est formée par réaction d'un anhydride d'acide carbonique comme étape préalable du ligand et des groupements amino de la matière de support T, et le ligand multimodal disposant d'au moins un groupement d'acide carboxylique libre.

**8.** Utilisation du milieu d'adsorption multimodal selon l'une des revendications 1 à 5 pour le nettoyage de biomolécules, les biomolécules étant des protéines, des peptides, des acides aminés, des acides nucléiques, des virus, des pseudo-particules virales et/ou des endotoxines.

**9.** Utilisation selon la revendication 8, les protéines étant des anticorps.

Fig. 1

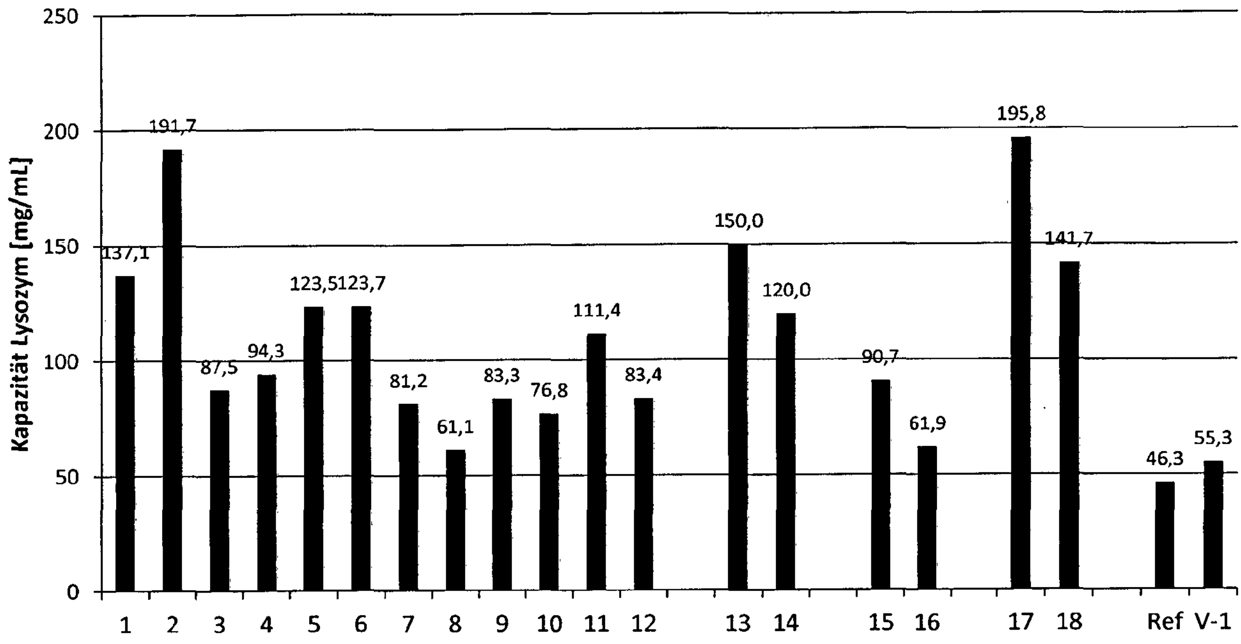
Kapazität Lysozym [mg/mL]
250
200
150
100
50
0
137,1
191,7
87,5
94,3
123,5
123,7
81,2
61,1
83,3
76,8
111,4
83,4
150,0
120,0
90,7
61,9
195,8
141,7
46,3
55,3
1 2 3 4 5 6 7 8 9 10 11 12 13 14 15 16 17 18 Ref V-1

Fig. 2

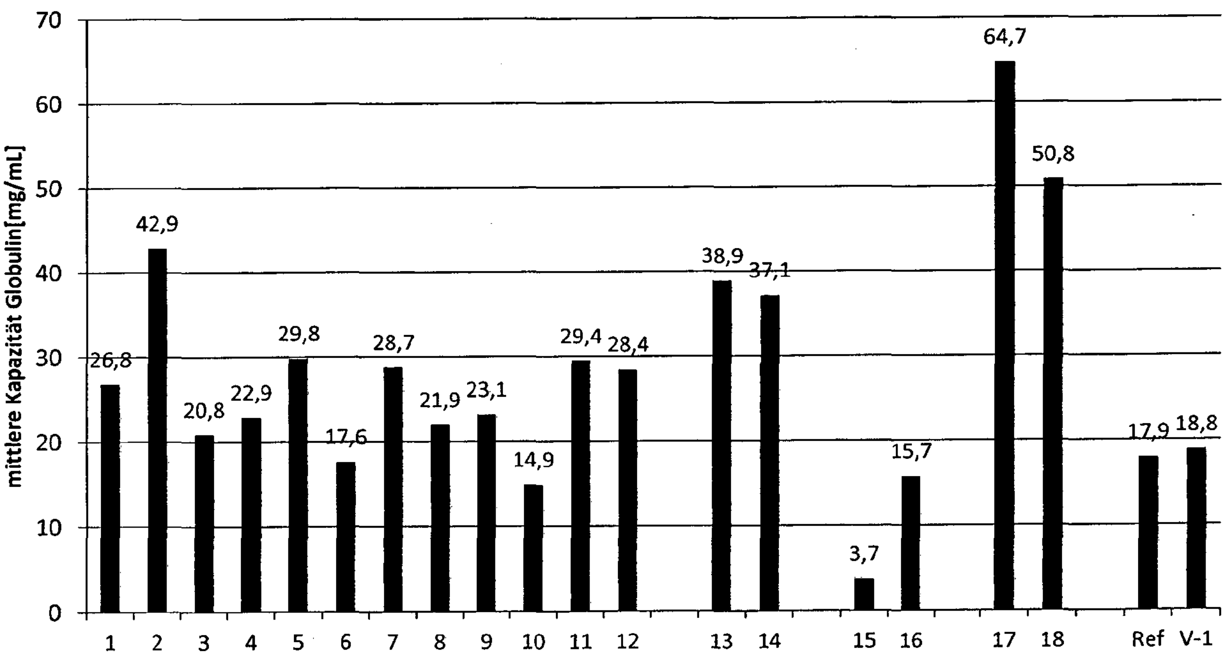
mittlere Kapazität Globulin[mg/mL]
70
60
50
40
30
20
10
0
26,8
42,9
20,8
22,9
29,8
17,6
28,7
21,9
23,1
14,9
29,4
28,4
38,9
37,1
3,7
15,7
64,7
50,8
17,9
18,8
1 2 3 4 5 6 7 8 9 10 11 12 13 14 15 16 17 18 Ref V-1

Fig. 3

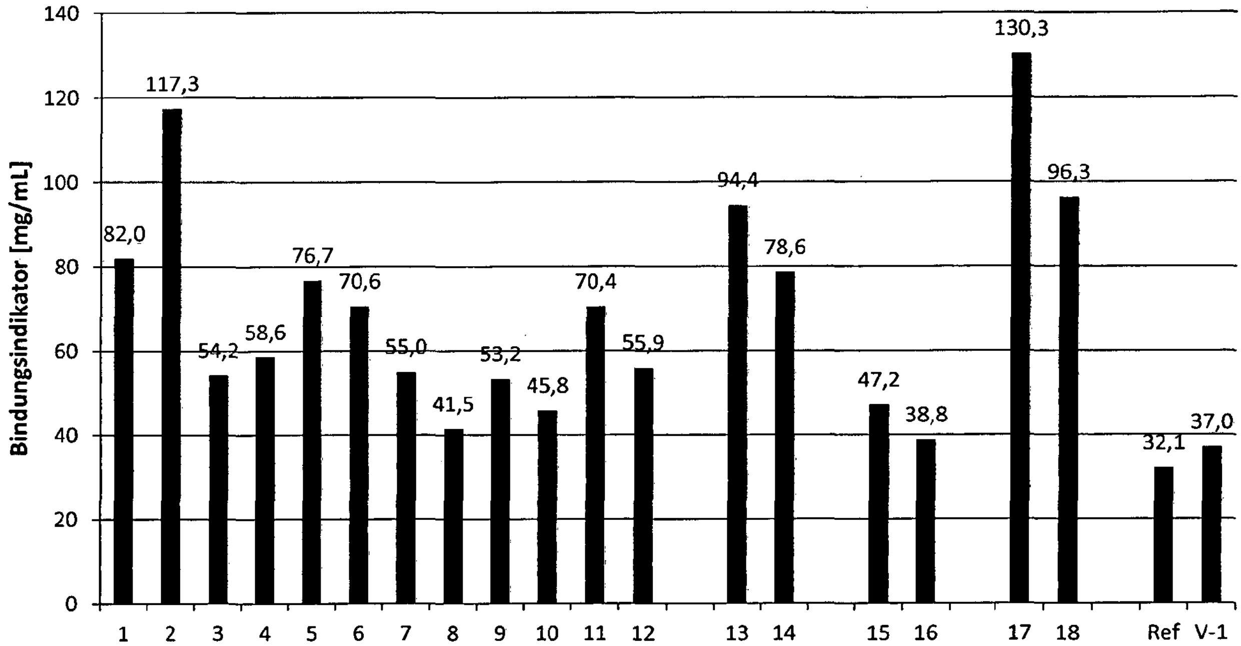
Bindungsindikator [mg/mL]
140
120
100
80
60
40
20
0
82,0
117,3
54,2
58,6
76,7
70,6
55,0
41,5
53,2
45,8
70,4
55,9
94,4
78,6
47,2
38,8
130,3
96,3
32,1
37,0
1 2 3 4 5 6 7 8 9 10 11 12 13 14 15 16 17 18 Ref V-1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5431807 A
- EP 0665867 B1
- US 20120202976 A1
- US 8877904 B2
- US 8017740 B2
- US 20130109807 A1
- US 6852230 B2
- WO 2006081002 A2
- DE 102008055821 A1


### In der Beschreibung aufgeführte Nicht-Patentliteratur


- **GREG T. HERMANSON ; A. KRISHNA MALLIA ; PAUL K. SMITH.** Immobilized Affinity Ligand Techniques. Academic Press, INC, 1992
- **B.-L. JOHANSSON et al.** *Journal of Chromatography A,* 2003, vol. 1016, 35-49